# EUROPEAN PATENT SPECIFICATION

(11) **EP 4 646 245 B1**
(45) Date of publication and mention of the grant of the patent: **07.10.2026**
(21) Application number: 23833804.0
(22) Date of filing: 19.12.2023
(51) Int. Cl.: A61M 5/148, A61M 5/155, A61M 5/168, A61M 5/172, A61M 5/44, A61M 5/142, A61M 5/145

(54) **MEDICAMENT DELIVERY SYSTEM FOR WARMING A MEDICAMENT WITH AIR AND METHOD THEREFOR**
MEDIKAMENTENABGABESYSTEM ZUM ERWÄRMEN EINES MEDIKAMENTS MITTELS LUFT UND VERFAHREN DAFÜR
SYSTÈME D'ADMINISTRATION DE MÉDICAMENT POUR CHAUFFER UN MÉDICAMENT À L'AIDE D'AIR ET PROCÉDÉ ASSOCIÉ

(30) Priority: 02.01.2023 US 202363436672 P; 02.01.2023 US 202363436677 P; 02.03.2023 EP 23159773
(43) Date of publication of application: 12.11.2025
(73) Proprietor: SHL Medical AG, 6302 Zug (CH)
(72) Inventor: SÄLL, Daniel, 131 28 Nacka Strand (SE); MYRMAN, Mattias, 131 28 Nacka Strand (SE); BALKANDJIEV, Plamen, 131 28 Nacka Strand (SE); NEBY, Torbjörn, 131 28 Nacka Strand (SE)
(86) International application number: PCT/EP2023/086569
(87) International publication number: WO 2024/146775

(56) References cited:
- US-A1- 2006 015 084
- US-A1- 2021 154 401
- US-A1- 2022 409 807
- US-B1- 7 351 226

## Description

### TECHNICAL FIELD

The present disclosure relates to the field of medicament delivery systems.

### BACKGROUND

Some drugs are required to be stored in a refrigerator for reasons of storage life and/or for the medicament not to lose its properties. However, in general and also for automatic and semi-automatic delivery systems, the drug should typically reach ambient temperature to render the delivery more comfortable since the injection of a cold substance can be painful. Moreover, the temperature also affects the viscosity for some medicaments.

In a medicament delivery system, the patient should typically let the drug warm at room temperature for about 30 minutes. However, depending on the volume of the medicament and the packaging, this step might take longer than expected or desired.

One conventional way to measure the temperature to know when the drug is ready for injection is to put some type of electronics in or close to the medicament or some type of temperature sensitive label on the medicament container. However, these solutions will add cost and complexity and also negatively affect the sustainability.

US2021/154401A1 relates to a device for the administration of liquids, drugs or nutrients to a patient. US7351226B1 relates to a medical infusion pump. US2022/409807A1 relates to a medicine injection device. US2006/015084A1 relates to a liquid dispenser.

It is thus an object of the present disclosure to remedy at least some of the deficiencies of the prior art.

### SUMMARY

According to an aspect, a (partially) reusable medicament delivery device/system is provided.

According to an aspect, a warm-up time of the drug may be reduced. According to an aspect, medicament delivery may be more comfortable for a patient.

According to an aspect, erroneous or incorrect medicament delivery may be prevented.

According to an aspect, a medication comprising the sequential dispensing of multiple drugs may be facilitated.

Moreover, methods and systems according to the present disclosure are provided to speed up the warming up of the drug as well as to measure the temperature of the drug and indicate when it is ready for injection.

The invention is specified by the independent claims. Preferred embodiments are defined in the dependent claims. In the following description, although numerous features may be designated as optional, it is nevertheless acknowledged that all features comprised in the independent claims are not to be read as optional.

The present invention relates to a medicament delivery system comprising a cassette comprising a medicament bag in an interior thereof, a pump unit separable from the cassette, wherein an air flow connection is provided between the pump unit and the interior of the cassette, wherein the pump unit comprises a pump and an air valve, wherein the pump is configured to pressurise the interior of the cassette to exert a pressure on the medicament bag, and wherein the air valve is configured to de-pressurise the cassette by releasing the pressure from the interior of the cassette and wherein the pump unit comprises at least one temperature sensor, wherein the at least one temperature sensor is provided in an air flow path at the pump and/or the air valve and/or an air filter connected to the pump.

Various embodiments may preferably implement the following features. Preferably, the pump unit is independent from said cassette.

Preferably, the cassette comprises an entry passage configured to communicate with a connector of the pump unit.

Preferably, the pump is configured to pressurise the cassette via the connector and the entry passage.

Preferably, the connector comprises a sealing member configured to seal the connection between the connector and the entry passage in an air-tight manner.

Preferably, the cassette comprises at least one outer shell and a lid, wherein the entry passage is preferably provided in the lid.

Preferably, the medicament bag is provided inside the at least one outer shell and the lid.

Preferably, the medicament bag is a flexible medicament bag.

Preferably, the cassette and the pump unit are connectable to each other via a slide-on connection.

Preferably, the cassette comprises a cassette locking mechanism and the pump unit comprises a corresponding pump unit locking mechanism configured to secure the cassette and the pump unit with respect to each other.

Preferably, one of the cassette locking mechanism and the pump unit locking mechanism comprises a guide rail, and wherein the other one of the cassette locking mechanism and the pump unit locking mechanism comprises a corresponding guide groove.

Preferably, the guide rail has a substantially T-shaped cross section.

The pump unit comprises at least one temperature sensor. Preferably the pump unit comprises at least one pressure sensor, wherein the at least one pressure sensor is/are preferably provided in an air flow path at the pump and/or the air valve.

Preferably, the at least one temperature sensor is configured to measure at least one of an ambient air temperature and a temperature of the interior of the cassette.

Preferably, the at least one pressure sensor is/are configured to measure the pressure of the interior of the cassette.

Preferably, the pump unit comprises a processing unit.

Preferably, the processing unit comprises a processor, a memory and a communication interface.

Preferably, the processing unit is configured to control at least one of the pump, the air valve, the indicator, or the communication interface.

Preferably, the pump unit comprises a connection switch configured to switch on the processing unit if a cassette is attached to the pump unit. Preferably, the pump unit comprises a power switch configured to activate the pump.

Preferably, the processing unit is configured to control the pump based on an ambient air temperature or a temperature of the interior of the cassette or a difference of the ambient air temperature and the temperature of the interior of the cassette.

Preferably, the processing unit is configured to control the pump and the air valve to periodically pressurise and de-pressurise the interior of the cassette.

Preferably, the processing unit is configured to perform the periodic pressurising and de-pressurising based on an ambient air temperature or a temperature of the interior of the cassette or a difference of the ambient air temperature and the temperature of the interior of the cassette.

Preferably, the pump unit comprises an air filter, wherein the pump is configured to suck in air through said air filter.

Preferably, a temperature sensor is provided at the air filter.

Preferably, the pump unit is reusable.

Preferably, the cassette is sealed in an air-tight manner apart from the entry passage.

Preferably, the pump unit comprises a release button configured to release the detachable pump unit from the cassette.

The present disclosure further relates to a pump unit comprising a pump and an air valve, wherein the pump unit is configured for a system as described above.

Various embodiments may preferably implement the following features. Preferably, the pump unit comprises a connector in fluid connection with the pump.

Preferably, the connector comprises a sealing member.

Preferably, the pump unit comprises a pump unit locking mechanism.

Preferably, the pump unit locking mechanism comprises a guide rail or a guide groove.

Preferably, the guide rail has a substantially T-shaped cross section.

Preferably, the pump unit comprises at least one pressure sensor, wherein the at least one pressure sensor is/are preferably provided in an air flow path at the pump and/or the air valve.

Preferably, the at least one temperature sensor is configured to measure at least one of an ambient air temperature and a temperature of the interior of the cassette.

Preferably, the pump unit comprises a processing unit.

Preferably, the processing unit comprises a processor, a memory and a communication interface.

Preferably, the processing unit is configured to control at least one of the pump, the air valve, the indicator, or the communication interface.

Preferably, the pump unit comprises a connection switch configured to switch on the processing unit if a cassette is attached to the pump unit.

Preferably, the pump unit comprises a power switch configured to activate the pump.

Preferably, the processing unit is configured to control the pump based on an ambient air temperature or another measured temperature or a difference of the ambient air temperature and the other measured temperature.

Preferably, the processing unit is configured to control the pump and the air valve to be periodically driven.

Preferably, the processing unit is configured to perform the periodic driving based on an ambient air temperature or another measured temperature or a difference of the ambient air temperature and the other measured temperature.

Preferably, the pump unit comprises an air filter, wherein the pump is configured to suck in air through said air filter.

Preferably, a temperature sensor is provided at the air filter.

Preferably, the pump unit is reusable.

Preferably, the pump unit comprises a release button.

The present disclosure further encompasses a cassette comprising a medicament bag in an interior thereof, wherein the cassette is configured for a medicament delivery system as described above.

Various embodiments may preferably implement the following features. Preferably, the cassette comprises an entry passage in fluid connection with the interior of the cassette.

Preferably, the cassette further comprises at least one outer shell and a lid, wherein the entry passage is preferably provided in the lid.

Preferably, the medicament bag is provided inside the at least one outer shell and the lid.

Preferably, the medicament bag is a flexible medicament bag.

Preferably, the cassette comprises a cassette locking mechanism.

Preferably, the cassette locking mechanism comprises a guide rail or a guide groove.

Preferably, the guide rail has a substantially T-shaped cross section. Preferably, the cassette is sealed in an air-tight manner apart from the entry passage.

The present disclosure further relates to a method for controlling a medicament delivery system as described above, the system comprising a cassette comprising a medicament bag and a pump unit detachably provided at the cassette. The method comprises pressurising the cassette to exert a pressure on the medicament bag, releasing a content of the medicament bag by the pressurising step, and de-pressurising the cassette.

The present invention further relates to a method for warming a medicament in a medicament delivery system, wherein the method comprises pressurising a cassette containing a medicament bag, and releasing, after a predetermined period of time, the pressurised air from the cassette.

Various embodiments may preferably implement the following features. Preferably, the method further comprises measuring an outflow temperature of the released air.

Preferably, the method further comprises repeating the pressurising and releasing step depending on the measured outflow temperature.

Preferably, the method further comprises measuring an inflow temperature and calculating a temperature difference between the inflow temperature and the outflow temperature.

Preferably, the method further comprises repeating the pressurising and releasing step depending on the measured temperature difference.

In the present disclosure, when the term "distal direction" is used, this refers to the direction pointing away from the dose delivery site during use of the medicament delivery device. When the term "distal part/end" is used, this refers to the part/end of the delivery device, or the parts/ends of the members thereof, which during use of the medicament delivery device is/are located furthest away from the dose delivery site. Correspondingly, when the term "proximal direction" is used, this refers to the direction pointing towards the dose delivery site during use of the medicament delivery device. When the term "proximal part/end" is used, this refers to the part/end of the delivery device, or the parts/ends of the members thereof, which during use of the medicament delivery device is/are located closest to the dose delivery site.

Further, the terms "longitudinal", "longitudinally", "axially" and "axial" refer to a direction of a longitudinal extension of the device which, in the figures, extends along the x-axis.

Similarly, the terms "lateral", "laterally", "transverse", "transversal" and "transversally" refer to a direction generally perpendicular to the longitudinal direction (x-axis) which is marked as the y-axis in the figures.

The terms "vertical" and "vertically" refer to a direction perpendicular to a plane spanned by the longitudinal and lateral direction (x-y-plane) which is marked as the z-axis in the figures.

Further, the terms "circumference", "circumferential", or "circumferentially" refer to a circumference or a circumferential direction relative to an axis. Similarly, "radial" or "radially" refer to a direction extending radially relative to the axis, and "rotation", "rotational" and "rotationally" refer to rotation relative to the axis.

The term "separable" in accordance with the present invention means that the pump unit can be physically separated from the cassette. A separable pump unit can also be carried on or affixed to the cassette.

The term "independent" in accordance with certain embodiments of the present invention means that the pump unit is functionally independent from the cassette and from the medicament bag(s). The pump unit according to the present invention can thus co-operate with a plurality of different cassettes, as desired by the user, wherein the cassette is adapted to communicate with a pump unit. Thus, the user is provided with several options as regards the use of the pump unit. If desired, however, the independence of the pump unit can be deactivated or disabled or at least restricted so that a given pump unit is operable with only one or a limited number of cassettes.

Generally, all terms used in the claims are to be interpreted according to their ordinary meaning in the technical field, unless explicitly defined otherwise herein. All references to a/an/the element, apparatus, member, component, means, etc. are to be interpreted openly as referring to at least one instance of the element, apparatus, member component, means, etc., unless explicitly stated otherwise.

### BRIEF DESCRIPTION OF THE DRAWINGS

Embodiments of the present disclosure will now be described by way of example only and with reference to the following accompanying drawings.
Figure 1A shows an embodiment of a medicament delivery system according to the present disclosure in a pre-assembly state.
Figure 1B shows the medicament delivery system according to Figure 1A in an assembly state.
Figure 1C shows the medicament delivery system according to Figure 1A or 1B in an assembled state.
Figure 2A shows a sectional view of a cassette according to an embodiment of the present disclosure.
Figure 2B shows another sectional view of the cassette according to Figure 2A.
Figures 3A, 3B and 3C show perspective views of an interior of a pump unit according to an embodiment of the present disclosure.
Figures 4A and 4B show detailed partial views of a locking mechanism according to an embodiment of the present disclosure.
Figure 5 shows a sectional view of a locking mechanism according to an embodiment of the present disclosure.
Figures 6A and 6B show partial views of a connection switch according to an embodiment of the present disclosure.
Figure 7 shows a sectional view of a connection between a cassette and a pump unit according to an embodiment of the present disclosure.
Figure 8 shows a sectional view schematically illustrating an air flow path according to an embodiment of the present disclosure.
Figure 9 shows a flow chart of an embodiment of the present disclosure.
Figure 10 shows a flow chart of an embodiment of the present disclosure.

### DETAILED DESCRIPTION

All elements and embodiments described below with reference to the drawings may be combined unless indicated otherwise.

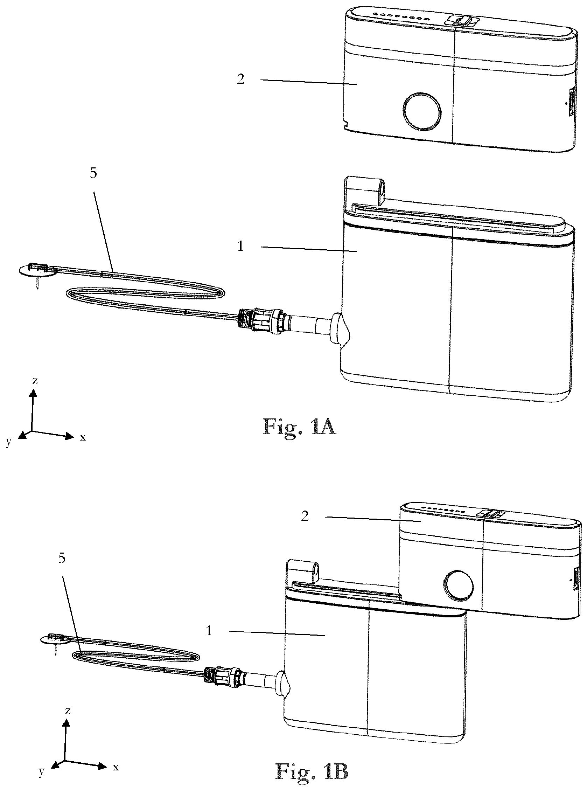

Fig. 1A shows an overview of a medicament delivery system according to an embodiment of the present disclosure. The system comprises a pump unit 2 and a cassette 1. The cassette 2 may be connectable to a tubing set 5. For example, a standard Luer connection may be employed. However, other types of tubing sets and connections can be used with the present disclosure. The cassette 1 and the pump unit 2 may be separately provided and connectable to each other.

Fig. 1B shows the pump unit 2 and the cassette 1 (with a tubing set 5 attached) during assembly or connection of both parts. The pump unit 2 is slid onto the cassette 1 or vice versa. In Fig. 1B, a sliding engagement between the pump unit 2 and the cassette 1 is shown but this is merely an example and not to be understood as limiting the disclosure.

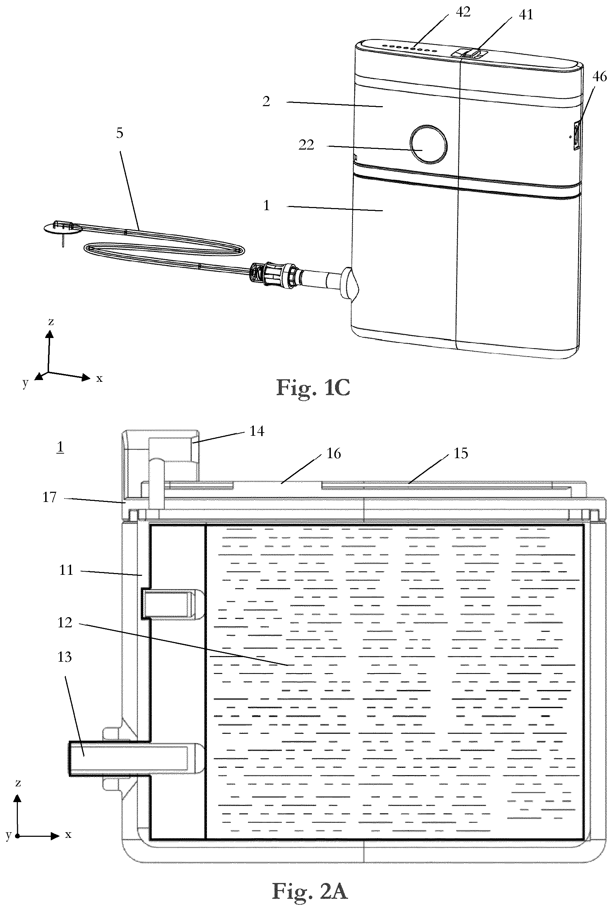

Fig. 1C shows the medicament delivery system in an assembled state. The pump unit 2 has been attached to the cassette 1 and both parts are locked with respect to each other. The device is thus ready for medicament delivery.

After medicament delivery, the pump unit 2 can be detached or separated from the cassette by pushing the release button 22 on the pump unit 2.

The pump unit 2 may thus be separable from the cassette 1. In particular, the pump unit 2 may be functionally independent from the cassette 1 and from the medicament bag(s) 12. The pump unit 2 according to the present invention can thus co-operate with a plurality of different cassettes 1, as desired by the user, wherein the cassette 1 is adapted to communicate with a pump unit 2. The pump unit 2 may also be configured to be connectable to only one (type of) cassette 1.

In a top view, both the pump unit 2 and the cassette 1 may have an oval shape. In particular, the pump unit 2 and the cassette 1 may have a similar or the same shape in an x-y-plane. In the assembled state, the circumferences of the pump unit 2 and the cassette 1 may be flush with respect to each other.

The vertical extension of the pump unit 2 and cassette 1 may differ depending on the electronics and parts provided in the pump unit 2 and the kind and volume of drug (i.e., the medicament bag 12) provided in the cassette 1. Various parts of the system will now be described in more detail.

Fig. 2A shows a sectional view of a cassette 1 according to an embodiment along a longitudinal direction (x-axis). The cassette 1 comprises an outer shell 11 which may be formed as a single hard shell or as a plurality of shell parts assembled together. The outer shell 11 or the shell parts may be closed by a cassette lid 17. Inside the outer shell 11, a medicament bag 12 containing a medicament (drug) is provided. The medicament may be liquid, in particular at the time of dispensing. The medicament bag 12 may be a flexible (collapsible) medicament bag 12. A volume of the medicament bag may be, e.g., between 10 ml and 250 ml, preferably between 50 ml and 100 ml. The medicament bag 12 has an outlet port 13 which may also be used for filling the medicament bag 12 during production. The outlet port 13 may reach through a respective opening in the outer shell 11. The outlet port 13 may be provided with a valve, such as a pinch valve, to allow medicament to flow out only if the valve is opened, e.g., by attaching a tubing set 5. The cassette 1 may further comprise an entry passage 14 allowing a fluid, in particular air, to enter the inside of the cassette 1.

The cassette 1 and the medicament bag 12 may be single use units or may be refillable.

In order to attach and secure the pump unit 2 to the cassette 1, a cassette locking mechanism 15 may be provided. The locking mechanism 15 may be provided on the cassette lid 17. In the figures, a guide rail 15 of a slide-on mechanism is shown. However, the pump unit 2 and the cassette 1 may also be connectable by a snap-fit, clamping, screwing or any other suitable means of connection. The locking mechanism 15 may also comprise locking cut-out 16 to be described later.

The cassette 1 is, apart from the entry passage 14, provided in an air-tight manner. That is, the outer shell 11 or outer shell parts and the cassette lid 17 are connected such that the interior of the cassette 1 is sealed. Consequently, the opening through which the outlet port 13 passes is also sealed in order not to allow air to pass.

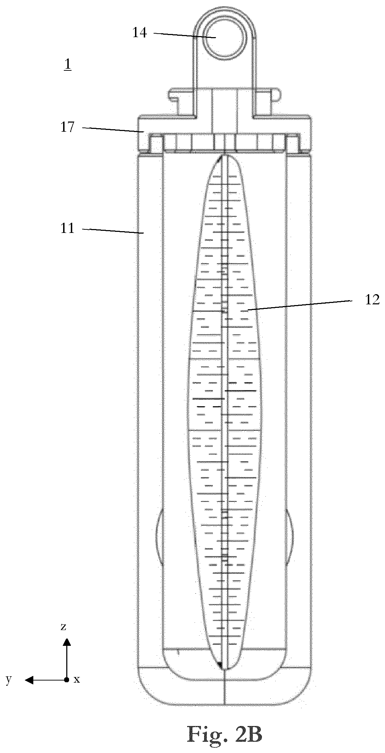

Fig. 2B is a further sectional view of the cassette according to Fig. 2A along a lateral direction (y-axis). As can be seen from the figure, between the outer shell 11, the cassette lid 17, i.e., the interior of the cassette 1, and the medicament bag 12, a space is provided. For medicament delivery, the interior of the cassette 1 is pressurised by introducing a fluid, in particular air, through the entry passage 14 to the space. Thereby, the pressure acts on the collapsible medicament bag 12 and forces the medicament through the outlet port 13 for medicament delivery. This process will be described in more detail below.

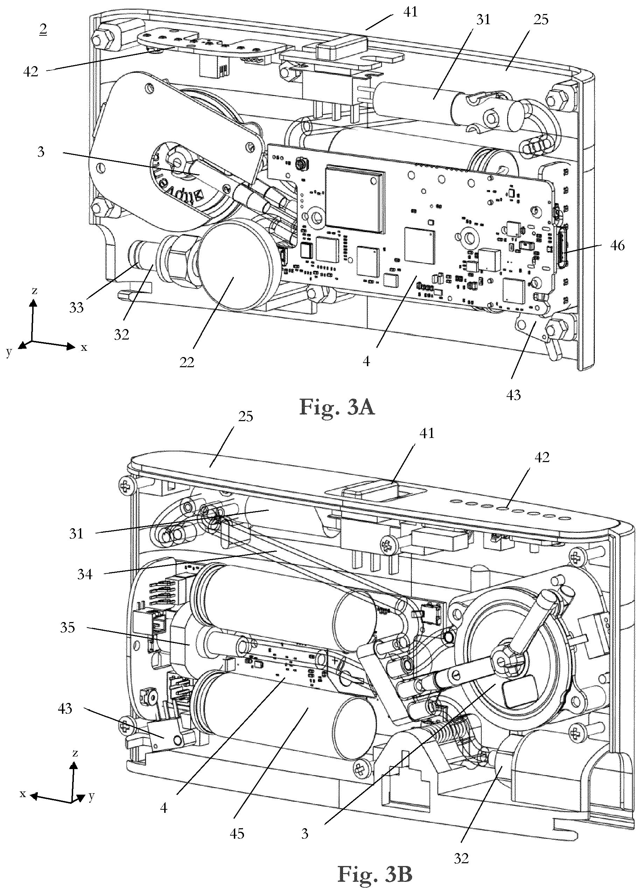

Fig. 3A is a perspective view of a pump unit 2 according to an embodiment. In the embodiments shown in the figures, the pump unit 2 comprises two shell parts 25 but it may also consist of one single shell part 25 or more than two shell parts 25. In Figs. 3A to 3C, the shell part 25 facing the viewer has been omitted to allow inspection of the interior.

The pump unit 2 comprises a pump 3, particularly an air pump 3, an air valve or pressure valve 31 and a connector 32 which serves as an air outlet for pressurising the cassette 1. The connector 32 may be provided with a sealing member 33. The sealing member 33 may be an O-ring fitted into a recess in the connector 32 as shown in Fig. 3A. The sealing member 33 may thus be radially compressed when a cassette 1 is attached to the pump unit 2 to seal the connection between the connector 32 and the entry passage 14 as will be further described below. The pump 3, the air valve 31 and the connector 32 may be connected by a tubing 34.

The pump unit 2 further comprises electronics such as a processing unit 4 for controlling the medicament delivery device. The processing unit 4 may also be referred to as a control unit. The processing unit 4 may comprise at least one processor, a memory, at least one power source 45 and a communication interface 46. The communication interface 46 may be used for charging the power source 45 and for connecting the pump unit 2 to a computer or a server. The pump unit 2 may, in addition or alternative to the power source 45, also comprise a disposable battery. In addition, a wireless communication module may be provided.

The pump unit 2 may also comprise an identification unit (not shown) configured to identify the type of cassette 1 attached to the pump unit 2, i.e., parameters or prescription particulars of the drug such as type, volume, temperature for delivery, speed of delivery, time of delivery, etc. Thus, the cassette 1 may comprise an identification tag allowing the pump unit 2 to identify the cassette 1. This may be achieved, e.g., via RFID, NFC or the like. It may be preferred that the identification tag at the cassette 1 is a passive component.

The processing unit 4 may control operation of the pump 3 and the air valve 31. Moreover, the processing unit 4 may be connected to a power switch 41 for starting a medicament delivery or switching the device on or off, and/or an indicator 42 for indicating various states of the device and a connection switch 43 for detection whether a cassette 1 is attached to the pump unit 2. The indicator 42 may comprise at least one illumination means such as an LED and/or a sound generator or speaker. The indicator 42 may provide visual and/or audible feedback of the state of the device which may comprise at least one of an active state of the processing unit 4, an attachment state of the cassette 1, an operational state, a battery state, a medicament temperature, a pressure state, an error state, a type of drug, whether the device is ready for injection, or whether the injection has been successful. The pump unit 2 may further comprise at least one pressure sensor and/or at least one temperature sensor (not shown). The pressure sensor may be provided at a suitable spot of the air flow path, e.g., at the connector 32, the pump 3, the air valve 31 or the tubing 34. The temperature may be measured, e.g., at the air filter 35, the connector 32, the pump 3 or the air valve 31 or in between at the tubing 34. Specifically, the temperature of the incoming air may be measured and/or the temperature of air flowing out of the cassette 1 in order to obtain data of a medicament temperature inside the cassette 1. Also, a medicament temperature may be directly measured.

Fig. 3B is a perspective view of the pump unit 2 from the other side of the device, i.e., rotated 180° around the vertical z-axis. Again, the shell part 25 facing the viewer has been omitted in the figure.

The pump unit 2 further comprises an air inlet which may comprise an air filter 35. The air inlet/air filter 35 is also connected to the pump 3 via the tubing 34.

A distribution unit may connect the pump 3, the air filter 34, the connector 32 and the air valve 31 with and via the tubing 34. The distribution unit may be a connector or an air switch. The distribution unit may also comprise a valve. In an embodiment, at least one of the air filter 34, the connector 32, or the air valve 31 may directly be connected to the pump 3.

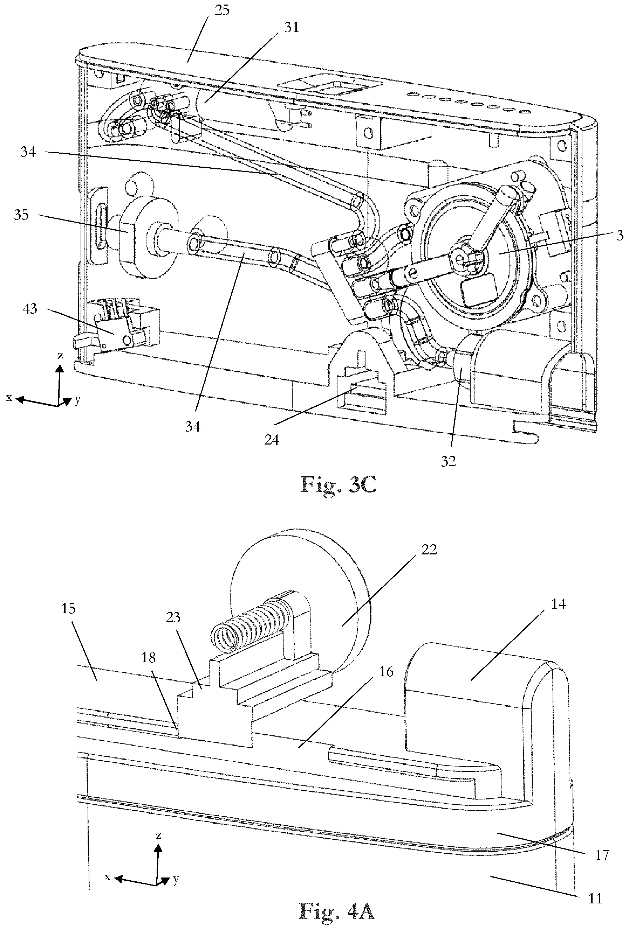

In Fig. 3C showing the same perspective view as Fig. 3B, most of the features apart from the air flow path have been omitted. In particular, ambient air is sucked in by the pump 3 via the air filter 35 and the tubing 34 and provided to an attached cassette 1 via the connector 32. During that process, the air valve 31 is closed in order to pressurise the cassette 1. When the pressure inside the cassette 1 is to be released, the air valve 31 is opened and the air flows to the outside via the tubing 34 and the air valve 31. The air inlet 35 and air outlet (i.e., the air valve 31) may be provided inside the pump unit 2 which may not be hermetically sealed. Alternatively, openings may be provided in the shell parts 25 allowing air to pass or connecting the air inlet 31 and the air outlet 35 to the outside.

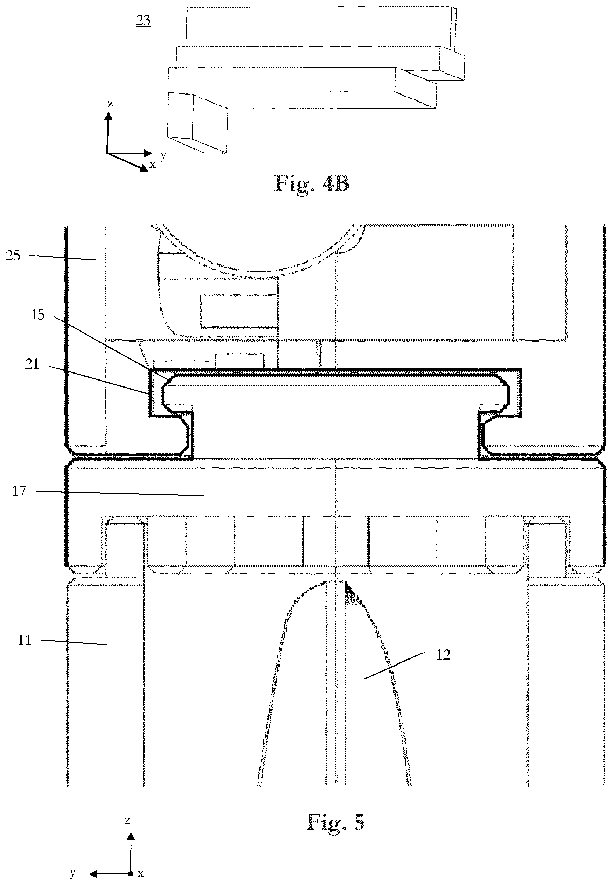

Referring to Figs. 4A, 4B and 5, an exemplary locking mechanism between the cassette 1 and the pump unit 2 according to an embodiment is described. As already described above, the cassette 1 may comprise a cassette locking mechanism 15 and the pump unit 2 may comprise a corresponding pump unit locking mechanism 21. In particular, the cassette unit locking mechanism 15 may be a guide rail and the pump unit locking mechanism 21 may be a guide groove. In an embodiment, the guide rail 15 may be a T-shaped rail and the guide groove 21 may be respectively recessed to receive the guide rail 15. The engagement of the guide rail 15 and the guide groove 21 can be seen from Fig. 5 showing a cross sectional view along the y-axis of the cassette 1 and the pump unit 2 in an attached state.

The locking mechanism between the pump unit 2 and the cassette 1 may also be provided vice versa, i.e., the pump unit locking mechanism 21 comprising a guide rail and the cassette unit locking mechanism 21 comprising a corresponding guide groove.

Herein, the longitudinal direction of insertion (negative x-direction) may be referred to as an insertion direction while the opposite longitudinal direction (positive x-direction) may be referred to as a release direction.

Referring to Fig. 4A, the guide rail 15 may comprise a locking cut-out 16 engaging a pump unit locking part 23. Other parts of the pump unit 2 have been omitted in this view to more clearly show the locking mechanism. The pump unit locking part 23 may be provided in a locking part guide groove 24 of the pump unit 2 or the shell part 25, respectively (see Fig. 3C). The locking part 23 may be connected to the release button 22 and movable in a lateral/transversal direction (y-axis) in the locking part guide groove 24 by actuation of the release button 22. The release button 22 may be provided with a spring acting on the locking part 23 and the release button 22 in the lateral direction. As can be seen from Fig. 4B, the locking part 23 may comprise a portion protruding in the negative z-direction (downwards in the figure) and configured to engage with the locking cut-out 16. The protruding portion may be sloped or inclined towards the direction of insertion (negative x-direction).

The attachment process may be as follows. The pump unit 2 is slid onto the cassette 1 in the longitudinal direction (negative x-direction, insertion direction) as shown in Fig. 1B via engagement of the guide rail 15 and the guide groove 21 (cf. Fig. 5). At the beginning of the sliding movement, the sloped portion of the locking part 23 abuts the guide rail 15, thus pushing the locking part 23 in the lateral direction. The release button 22 may also move in the lateral direction together with the locking part 23. The pump unit 2 and the cassette 1 are thus guided with respect to each other via the above engagement until the assembled state is reached. In the assembled state, the locking part 23 has fully passed the beginning of the locking cut-out 16 and is thus free to move in the lateral direction (y-direction) into the locking cut-out 16 by the force of the spring. A longitudinal end surface of the locking part 23 abuts an abutment surface 18 at a longitudinal end of the locking cut-out 16 in the release direction and thus prevents movement of the pump unit 2 with respect to the cassette 1 in said release direction. Moreover, in the assembled state, the connector 32 is in engagement with the entry passage 14, thus blocking further movement in the insertion direction (negative x-direction). This will be further described below. Reaching the assembled state may be notified to the patient or user by auditive and/or tactile feedback such as a click or vibration produced by the interaction of the locking part 23 and the locking cut-out 16.

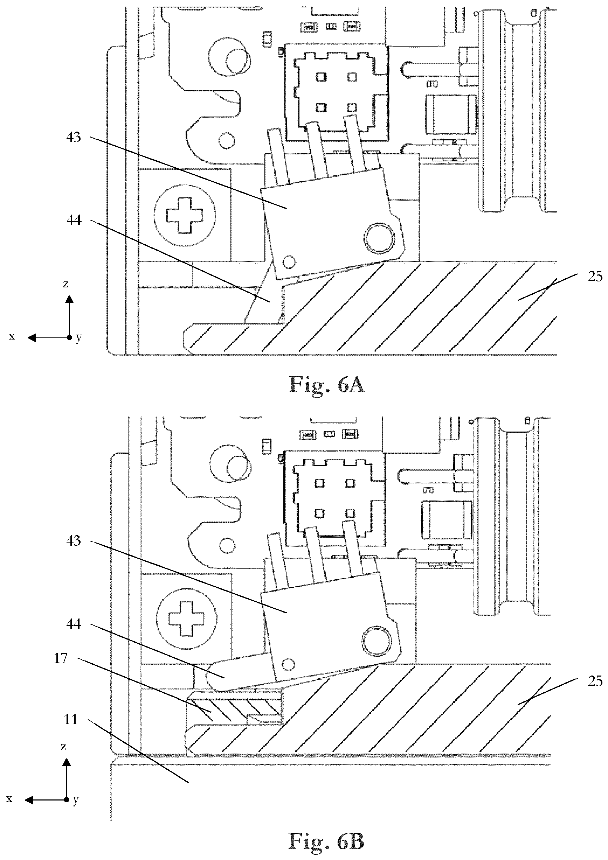

In addition, the pump unit 2, in particular the processing unit 4, may be activated by the connection switch 43 and switched from a sleep state to an active state. Therefore, the connection switch 43 may detect whether a cassette 1 is attached. The connection switch 43 may be provided at the longitudinal end of the pump unit 2 in the release direction and towards a lower end of the pump unit 2 (negative z-direction), i.e., towards the cassette 1. In the figures, a mechanical connection switch 43 comprising a switch part 44 is shown, but the connection switch 43 may also be an optical sensor, an inductive sensor or another electronic detection device. In Fig. 6A, the pump unit 2 is not or not fully attached to the cassette 1. The switch part 44 points in the negative-z direction (downwards in the figure). When the pump unit 2 is fully slid onto the cassette 1, a part of the cassette 1, in this case the lid 17 and the guide rail 15, respectively, lifts up the switch part 44 thus notifying to the processing unit 4 via the switch part 44 that the cassette 1 has been fully attached and the medicament delivery device is thus assembled for medicament delivery.

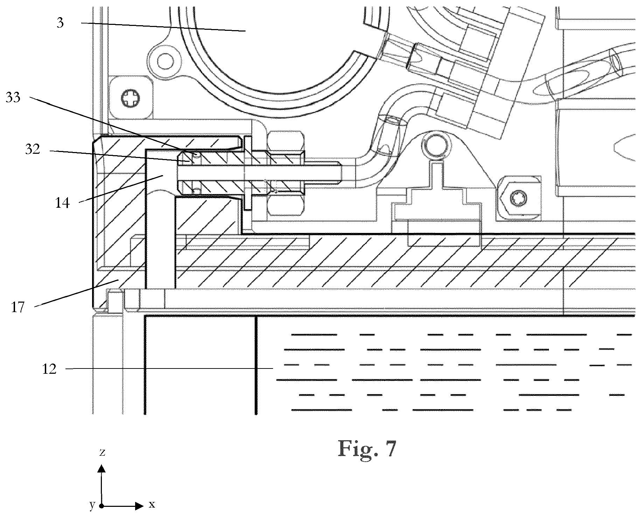

In Fig. 7, the air flow connection between the pump unit 2 and the cassette 1 is shown. The entry passage 14 may protrude in the vertical direction (z-direction) from the cassette 1 and the lid 17, respectively. The pump unit 2 or the shell part(s) 25 may comprise a respective receiving portion recessed or cut out from the shell part(s) 25 for receiving the connector 32. In particular, the connector 32 may be provided as a protruding rod protruding in the longitudinal direction (insertion direction) and may be slid into the entry passage 14. As indicated above, said connection shall be air-tight in order to allow pressurising the inside of the cassette 1 via the connector 32 using air provided by the pump 3. Hence, a sealing member 33 may be provided sealing the connection between the connector 32 and the entry passage 14. The sealing member 33 may be an O-ring or other suitable means of preventing air leakage. In case of an O-ring as shown in Fig. 7, a radial recess may be provided in the connector 32 in which the O-ring is fitted. When attaching the cassette 1 to the pump unit 2, the connector 32 is slid into the entry passage 14 and the sealing member 33 is radially compressed, thus sealing the connection between the cassette 1 and the pump unit 2.

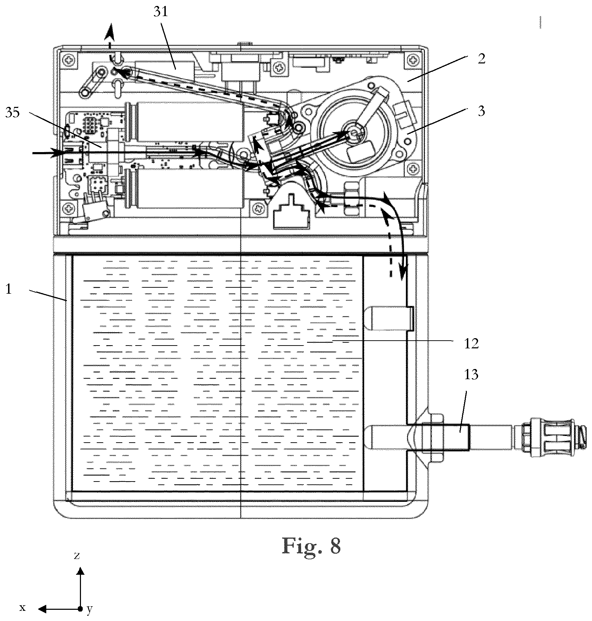

Fig. 8 shows the air flow inside the device according to an embodiment. Therein, the solid line indicates the inflow air, and the dashed line indicates the outflow air. For medicament delivery, ambient air is sucked in by the pump 3 through the air filter 35 and the tubing 34 and then provided to the cassette 1 via the tubing 34, the connector 32 and the entry passage 14. By pumping air into the cassette 1, the interior of the cassette 1 is pressurised, wherein the pressure acts on the medicament bag 12 to force the medicament into the tubing set. In other words, the pressure in the interior of the cassette 1 exerts a force on the medicament bag 12, in particular on the outer surface of the medicament bag 12. Thereby, the medicament is pushed out of the medicament bag 12 through the outlet port 13.

In case air is to be released from the cassette 1, the air valve 31 is opened, and the interior of the cassette 1 may be de-pressurised through the entry passage 14, the connector 32, the tubing 34 and the air valve 31.

The figures of the present disclosure refer to a slide-on mechanism between the pump unit 2 and the cassette 1. Such a slide-on mechanism may facilitate sealing of both units with respect to each other due to the engagement of the connector 32 and the entry passage 14. However, other mechanisms such as snap-fit, screw-on, or the like may be provided as long as an air-tight connection allowing the pump 3 to pressurise the cassette 1 is ensured. Also, air inlet and air outlet may be separately provided in the cassette 1.

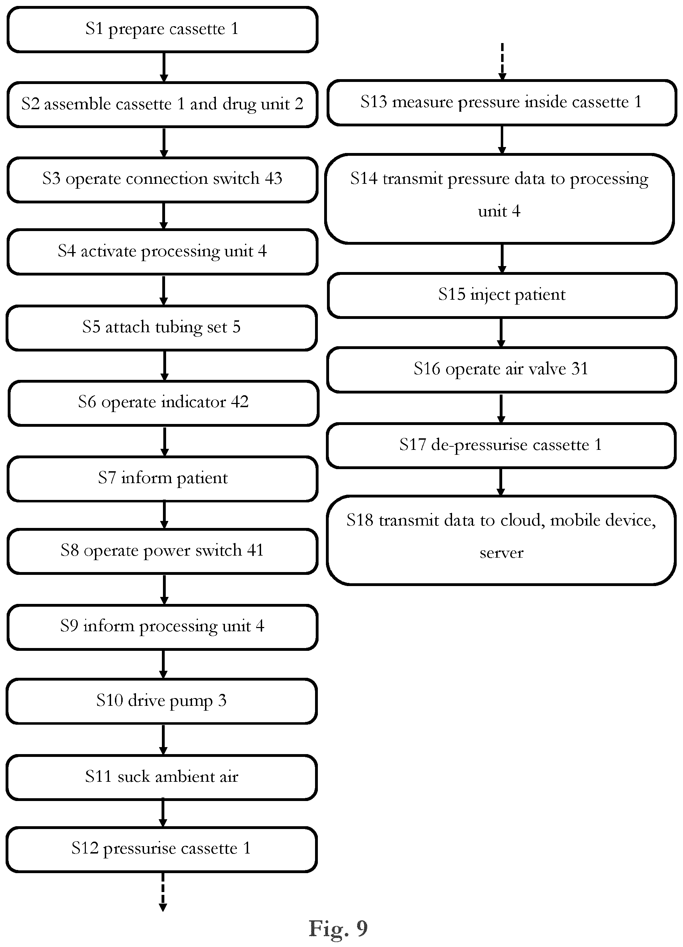

An operation of the medicament delivery system according to an embodiment will now be described. An exemplary flow chart of the method is depicted in Fig. 9.

A pharmacy may be instructed to prepare S1 a cassette 1 having a medicament bag 12 according to the prescription of the patient. Depending on the medicament, the cassette 1 may be stored in a cool and dark place such as a refrigerator. Thus, the patient may collect the cassette 1 from the refrigerator prior to delivery for acclimatisation and let it rest at room temperature for a period of time (e.g., 30 minutes).

The patient then assembles S2 the device by connecting the pump unit 2 to the cassette 1 as described above. The cassette 1 operates S3 the connection switch 43 thus activating S4 the processing unit 4 and notifying that a cassette 1 has been attached. The patient may now attach S5 an tubing set 5 to the cassette 1.

The processing unit 4 may operate S6 the indicator 42 to inform S7 the patient of the current state of the device. In particular, the indicator 42 may provide visual and/or audible feedback of the state of the device which may comprise at least one of an active state of the processing unit 4, an attachment state of the cassette 1, an operational state, a battery state, a medicament temperature, a pressure state, an error state, a type of drug, whether the device is ready for injection, or whether the injection has been successful.

When the indicator 42 indicates that the device is ready for injection, the patient may operate S8 the power switch 41 which transmits S9 a start signal informing the processing unit 4 to start or pause the injection.

The processing unit 4 drives S10 the air pump 3 for pressurising the cassette 1 and starting the injection. In case the patient pauses or stops the injection via the power switch 41, the air valve 31 (pressure valve) may be operated to release the pressure inside the cassette 1 and stop the drug flow.

When the injection is started, the pump 3 sucks S11 ambient air and pressurises S12 the cassette 1 with said ambient air as described above. A pressure sensor may measure S13 or sense a pressure inside the cassette 1 and transmit S14 the data to the processing unit 4. Consequently, the patient is injected S15 via the tubing set 5. The pump 3 raises the pressure inside the cassette 1. A maximum pressure may be limited by a pressure sensor that informs the processing unit 4. The pressure acts on the collapsible medicament bag 12 containing the drug. The pressurised drug is forced through the tubing set 5 and into the patient tissue via a needle. The amount of delivered dose may be controlled by time and cassette pressure. According to the delivery state, the processing unit 4 may operate S16 the air valve 31 and thereby de-pressurise S17 the cassette 1. When the dose is given, the processing unit 4 may operate the pressure valve 31 that releases the pressure from the cassette 1. The indicator 42 on the pump unit 2 may inform the patient that the injection is complete. The tubing set 5 may be removed from the body and may be disposed of together with the cassette 1 in a medical waste container. The pump unit 2 is reusable and can be stored until a next planned injection.

The processing unit 4 may further transmit S18 the injection parameters to a cloud, a mobile device, a computer, a server, or the like.

The method described above may be performed with a medicament delivery system as previously described.

As mentioned above, some drugs are required to be stored in a refrigerator. However, in general the drug should reach ambient temperature to render the delivery more comfortable since the injection of a cold substance can be painful. Moreover, the temperature also affects the viscosity for some medicaments.

In an embodiment, to shorten the time until the correct temperature has been reached, air is circulated in the cassette 1 by adding overpressure and then releasing it to the atmosphere by opening the pressure valve 31. This may be repeated several times to exchange the cooled off air (by heat exchange with the cold drug) with warmer ambient air from the surrounding of the pump unit 2. A temperature sensor in the air circuit (inside the pump unit 2) may measure a temperature of air flowing into the system via the air filter 35. The air may be provided to the cassette 1 as described above by the pump 3 via the tubing 34, the connector 32 and the entry passage 14. The pressurised air may be contained within the cassette 1 for a set time to warm up the drug. After the set time, the pressurised air may be released to the atmosphere via the tubing 34 by opening the air valve 31. A temperature of the exit air flow may be measured. Said temperature can be used as an indication of the temperature of the drug inside the medicament bag 12 in the cassette 1. In particular, calculations using the difference between the air inflow temperature and the air outflow temperature may be used to determine when the drug is ready to be injected. A patient may then be notified via the indicator 42 that it is possible to start the injection.

The shortening of the warm-up will be facilitated in three ways. Firstly, the exchange of air after a period of time, and taking in new (warm) air from the surroundings and expelling the cooler air, will accelerate the warm-up. Secondly, convection, i.e., the movement/circulation of air inside the cassette 1, will speed up the procedure of warming up the medicament bag 12. This happens at every air exchange but can also be done more frequently by shortly blowing in or releasing air. Thirdly, the air inside the cassette 1 may be warmed up by the raised pressure from the pump 3. That is, the heat transfer may be more efficient due to the higher air pressure against the medicament bag 12 holding the drug.

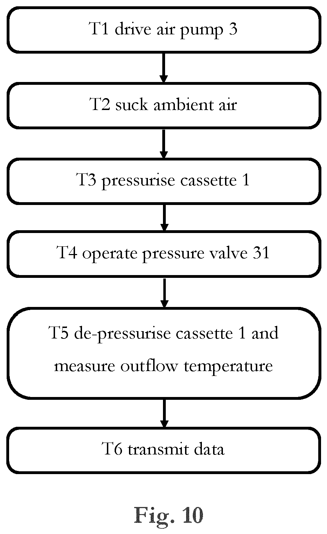

The process for an assembled device as described above may be performed as follows with reference to Fig. 10. The processing unit 4 may drive T1 the air pump 3 to suck T2 ambient air and pressurise T3 the interior of the cassette 1. The processing unit 4 may, after a predetermined period of time, operate T4 the air valve 31 to de-pressurise T5 the cassette 1 and release the air. Due to heat exchange with the cold drug, the released air will likely be colder than the ambient air. A temperature sensor measures, during de-pressurising T5, the temperature of the release air and transmits T6 the data to the processing unit 4. Depending on the measured temperature, the process T1 to T6 may be repeated until a pre-set temperature is reached or until a temperature difference between ambient air (inflow air) and exchanged air (outflow air) is below a certain threshold.

The method described above may be performed with a medicament delivery system as previously described and is also compatible with the previously described method.

The present disclosure may also encompass a pump unit 2 and/or a cassette 1 for use with the described system and/or methods. In particular, the present disclosure encompasses a pump unit 2 independent from a cassette 1.

The delivery devices described herein can be used for the treatment and/or prophylaxis of one or more of many different types of disorders.

Exemplary disorders include, but are not limited to: rheumatoid arthritis, inflammatory bowel diseases (e.g. Crohn's disease and ulcerative colitis), hypercholesterolaemia and/or dyslipidemia, cardiovascular disease, diabetes (e.g. type 1 or 2 diabetes), psoriasis, psoriatic arthritis, spondyloarthritis, hidradenitis suppurativa, Sjögren's syndrome, migraine, cluster headache, multiple sclerosis, neuromyelitis optica spectrum disorder, anaemia, thalassemia, paroxysmal nocturnal hemoglobinuria, hemolytic anaemia, hereditary angioedema, systemic lupus erythematosus, lupus nephritis, myasthenia gravis, Behçet's disease, hemophagocytic lymphohistiocytosis, atopic dermatitis, retinal diseases (e.g., age-related macular degeneration, diabetic macular edema), uveitis, infectious diseases, bone diseases (e.g., osteoporosis, osteopenia), asthma, chronic obstructive pulmonary disease, thyroid eye disease, nasal polyps, transplant, acute hypoglycaemia, obesity, anaphylaxis, allergies, sickle cell disease, Alzheimer's disease, Parkinson's disease, dementia with Lewy bodies, systemic infusion reactions, immunoglobulin E (IgE)-mediated hypersensitivity reactions, cytokine release syndrome, immune deficiencies (e.g., primary immunodeficiency, chronic inflammatory demyelinating polyneuropathy), enzyme deficiencies (e.g., Pompe disease, Fabry disease, Gaucher disease), growth factor deficiencies, hormone deficiencies, coagulation disorders (e.g., hemophilia, von Willebrand disease, Factor V Leiden), and cancer.

Exemplary types of drugs that could be included in the delivery devices described herein include, but are not limited to, small molecules, hormones, cytokines, blood products, enzymes, vaccines, anticoagulants, immunosuppressants, antibodies, antibody-drug conjugates, neutralizing antibodies, reversal agents, radioligand therapies, radioisotopes and/or nuclear medicines, diagnostic agents, bispecific antibodies, proteins, fusion proteins, peptibodies, polypeptides, pegylated proteins, protein fragments, nucleotides, protein analogues, protein variants, protein precursors, protein derivatives, chimeric antigen receptor T cell therapies, cell or gene therapies, oncolytic viruses, or immunotherapies.

Exemplary drugs that could be included in the delivery devices described herein include, but are not limited to, immuno-oncology or bio-oncology medications such as immune checkpoints, cytokines, chemokines, clusters of differentiation, interleukins, integrins, growth factors, coagulation factors, enzymes, enzyme inhibitors, retinoids, steroids, signaling proteins, pro-apoptotic proteins, anti-apoptotic proteins, T-cell receptors, B-cell receptors, or costimulatory proteins.

Exemplary drugs that could be included in the delivery devices described herein include, but are not limited to, those exhibiting a proposed mechanism of action, such as human epidermal growth factor receptor 2 (HER-2) receptor modulators, interleukin (IL) modulators, interferon (IFN) modulators, complement modulators, glucagon-like peptide-1 (GLP-1) modulators, glucose-dependent insulinotropic polypeptide (GIP) modulators, cluster of differentiation 38 (CD38) modulators, cluster of differentiation 22 (CD22) modulators, C1 esterase modulators, bradykinin modulators, C-C chemokine receptor type 4 (CCR4) modulators, vascular endothelial growth factor (VEGF) modulators, B-cell activating factor (BAFF), P-selectin modulators, neonatal Fc receptor (FcRn) modulators, calcitonin gene-related peptide (CGRP) modulators, epidermal growth factor receptor (EGFR) modulators, cluster of differentiation 79B (CD79B) modulators, tumor-associated calcium signal transducer 2 (Trop-2) modulators, cluster of differentiation 52 (CD52) modulators, B-cell maturation antigen (BCMA) modulators, enzyme modulators, platelet-derived growth factor receptor A (PDGFRA) modulators, cluster of differentiation 319 (CD319 or SLAMF7) modulators, programmed cell death protein 1 and programmed death-ligand 1 (PD-1/PD-L1) inhibitors/modulators, B-lymphocyte antigen cluster of differentiation 19 (CD19) inhibitors, B-lymphocyte antigen cluster of differentiation 20 (CD20) modulators, cluster of differentiation 3 (CD3) modulators, cytotoxic T-lymphocyte-associated protein 4 (CTLA-4) inhibitors, T-cell immunoglobulin and mucin-domain containing-3 (TIM-3) modulators, T cell immunoreceptor with Ig and ITIM domains (TIGIT) modulators, V-domain Ig suppressor of T cell activation (VISTA) modulators, indoleamine 2,3-dioxygenase (IDO or INDO) modulators, poliovirus receptor-related immunoglobulin domain-containing protein (PVRIG) modulators, lymphocyte-activation gene 3 (LAG3; also known as cluster of differentiation 223 or CD223) antagonists, cluster of differentiation 276 (CD276 or B7-H3) antigen modulators, cluster of differentiation 47 (CD47) antagonists, cluster of differentiation 30 (CD30) modulators, cluster of differentiation 73 (CD73) modulators, cluster of differentiation 66 (CD66) modulators, cluster of differentiation w137 (CDw137) agonists, cluster of differentiation 158 (CD158) modulators, cluster of differentiation 27 (CD27) modulators, cluster of differentiation 58 (CD58) modulators, cluster of differentiation 80 (CD80) modulators, cluster of differentiation 33 (CD33) modulators, cluster of differentiation 159 (CD159 or NKG2) modulators, glucocorticoid-induced TNFR-related (GITR) protein modulators, Killer Ig-like receptor (KIR) modulators, growth arrest-specific protein 6 (GAS6)/AXL pathway modulators, A proliferation-inducing ligand (APRIL) receptor modulators, human leukocyte antigen (HLA) modulators, epidermal growth factor receptor (EGFR) modulators, B-lymphocyte cell adhesion molecule modulators, cluster of differentiation w123 (CDw123) modulators, Erbb2 tyrosine kinase receptor modulators, endoglin modulators, mucin modulators, mesothelin modulators, hepatitis A virus cellular receptor 2 (HAVCR2) antagonists, cancer-testis antigen (CTA) modulators, tumor necrosis factor receptor superfamily, member 4 (TNFRSF4 or OX40) modulators, adenosine receptor modulators, inducible T cell co-stimulator (ICOS) modulators, cluster of differentiation 40 (CD40) modulators, tumor-infiltrating lymphocytes (TIL) therapies, or T-cell receptor (TCR) therapies. Exemplary drugs that could be included in the delivery devices described herein include, but are not limited to: etanercept, abatacept, adalimumab, evolocumab, exenatide, secukinumab, erenumab, galcanezumab, fremanezumab-vfrm, alirocumab, methotrexate (amethopterin), tocilizumab, interferon beta-1a, interferon beta-1b, peginterferon beta-1a, sumatriptan, darbepoetin alfa, belimumab, sarilumab, semaglutide, dupilumab, reslizumab, omalizumab, glucagon, epinephrine, naloxone, insulin, amylin, vedolizumab, eculizumab, ravulizumab, crizanlizumab-tmca, certolizumab pegol, satralizumab, denosumab, romosozumab, benralizumab, emicizumab, tildrakizumab, ocrelizumab, ofatumumab, natalizumab, mepolizumab, risankizumab-rzaa, ixekizumab, and immune globulins.

Exemplary drugs that could be included in the delivery devices described herein may also include, but are not limited to, oncology treatments such as ipilimumab, nivolumab, pembrolizumab, atezolizumab, durvalumab, avelumab, cemiplimab, rituximab, trastuzumab, ado-trastuzumab emtansine, fam-trastuzumab deruxtecan-nxki, pertuzumab, transtuzumab-pertuzumab, alemtuzumab, belantamab mafodotin-blmf, bevacizumab, blinatumomab, brentuximab vedotin, cetuximab, daratumumab, elotuzumab, gemtuzumab ozogamicin, 90-Yttrium-ibritumomab tiuxetan, isatuximab, mogamulizumab, moxetumomab pasudotox, obinutuzumab, ofatumumab, olaratumab, panitumumab, polatuzumab vedotin, ramucirumab, sacituzumab govitecan, tafasitamab, or margetuximab.

Exemplary drugs that could be included in the delivery devices described herein include "generic" or biosimilar equivalents of any of the foregoing, and the foregoing molecular names should not be construed as limiting to the "innovator" or "branded" version of each, as in the non-limiting example of innovator medicament adalimumab and biosimilars such as adalimumab-afzb, adalimumab-atto, adalimumab-adbm, and adalimumab-adaz. Exemplary drugs that could be included in the delivery devices described herein also include, but are not limited to, those used for adjuvant or neoadjuvant chemotherapy, such as an alkylating agent, plant alkaloid, antitumor antibiotic, antimetabolite, or topoisomerase inhibitor, enzyme, retinoid, or corticosteroid. Exemplary chemotherapy drugs include, by way of example but not limitation, 5-fluorouracil, cisplatin, carboplatin, oxaliplatin, doxorubicin, daunorubicin, idarubicin, epirubicin, paclitaxel, docetaxel, cyclophosphamide, ifosfamide, azacitidine, decitabine, bendamustine, bleomycin, bortezomib, busulfan, cabazitaxel, carmustine, cladribine, cytarabine, dacarbazine, etoposide, fludarabine, gemcitabine, irinotecan, leucovorin, melphalan, methotrexate, pemetrexed, mitomycin, mitoxantrone, temsirolimus, topotecan, valrubicin, vincristine, vinblastine, or vinorelbine.

Exemplary drugs that could be included in the delivery devices described herein also include, but are not limited to, analgesics (e.g., acetaminophen), antipyretics, corticosteroids (e.g. hydrocortisone, dexamethasone, or methylprednisolone), antihistamines (e.g., diphenhydramine or famotidine), antiemetics (e.g., ondansetron), antibiotics, antiseptics, anticoagulants, fibrinolytics (e.g., recombinant tissue plasminogen activator [r-TPA]), antithrombolytics, or diluents such as sterile water for injection (SWFI), 0.9% Normal Saline, 0.45% normal saline, 5% dextrose in water, 5% dextrose in 0.45% normal saline, Lactated Ringer's solution, Heparin Lock Flush solution, 100 U/mL Heparin Lock Flush Solution, or 5000 U/mL Heparin Lock Flush Solution.

Pharmaceutical formulations including, but not limited to, any drug described herein are also contemplated for use in the delivery devices described herein, for example pharmaceutical formulations comprising a drug as listed herein (or a pharmaceutically acceptable salt of the drug) and a pharmaceutically acceptable carrier. Such formulations may include one or more other active ingredients (e.g., as a combination of one or more active drugs), or may be the only active ingredient present, and may also include separately administered or co-formulated dispersion enhancers (e.g. an animal-derived, human-derived, or recombinant hyaluronidase enzyme), concentration modifiers or enhancers, stabilizers, buffers, or other excipients. Exemplary drugs that could be included in the delivery devices described herein include, but are not limited to, a multi-medication treatment regimen such as AC, Dose-Dense AC, TCH, GT, EC, TAC, TC, TCHP, CMF, FOLFOX, mFOLFOX6, mFOLFOX7, FOLFCIS, CapeOx, FLOT, DCF, FOLFIRI, FOLFIRINOX, FOLFOXIRI, IROX, CHOP, R-CHOP, RCHOP-21, Mini-CHOP, Maxi-CHOP, VR-CAP, Dose-Dense CHOP, EPOCH, Dose-Adjusted EPOCH, R-EPOCH, CODOX-M, IVAC, HyperCVAD, R-HyperCVAD, SC-EPOCH-RR, DHAP, ESHAP, GDP, ICE, MINE, CEPP, CDOP, GemOx, CEOP, CEPP, CHOEP, CHP, GCVP, DHAX, CALGB 8811, HIDAC, MOpAD, 7 + 3, 5 +2, 7 + 4, MEC, CVP, RBAC500, DHA-Cis, DHA-Ca, DHA-Ox, RCVP, RCEPP, RCEOP, CMV, DDMVAC, GemFLP, ITP, VIDE, VDC, VAI, VDC-IE, MAP, PCV, FCR, FR, PCR, HDMP, OFAR, EMA/CO, EMA/EP, EP/EMA, TP/TE, BEP, TIP, VIP, TPEx, ABVD, BEACOPP, AVD, Mini-BEAM, IGEV, C-MOPP, GCD, GEMOX, CAV, DT-PACE, VTD-PACE, DCEP, ATG, VAC, VeIP, OFF, GTX, CAV, AD, MAID, AIM, VAC-IE, ADOC, or PE.

Various modifications to the embodiments described are possible and will occur to those skilled in the art without departing from the invention which is defined by the following claims.

## Claims

1. Medicament delivery system comprising:
a cassette (1) comprising a medicament bag (12) in an interior thereof,
a pump unit (2) separable from the cassette (1), wherein an air flow connection is provided between the pump unit (2) and the interior of the cassette (1),
wherein the pump unit (2) comprises a pump (3) and an air valve (31),
wherein the pump (3) is configured to pressurise the interior of the cassette (1) to exert a pressure on the medicament bag (12), and
wherein the air valve (31) is configured to de-pressurise the cassette (1) by releasing the pressure from the interior of the cassette (1)
**characterized in that** the pump unit (2) comprises at least one temperature sensor, wherein the at least one temperature sensor is provided in an air flow path at the pump (3) and/or the air valve (31) and/or an air filter (35) connected to the pump (3).

2. Medicament delivery system according to claim 1, wherein the pump unit (2) comprises at least one pressure sensor, wherein the at least one pressure sensor is preferably provided in an air flow path at the pump (3) and/or the air valve (31) and/or an air filter (35) connected to the pump (3).

3. Medicament delivery system according to claim 1, wherein the at least one temperature sensor is configured to measure at least one of an ambient air temperature and a temperature of the interior of the cassette (1).

4. Medicament delivery system according to claim 1, 2 or 3, wherein the at least one pressure sensor is/are configured to measure the pressure of the interior of the cassette (1).

5. Medicament delivery system according to any one of claims 1 to 4, wherein the pump unit (2) comprises a processing unit (4) and wherein the processing unit (4) is configured to control the pump (3) based on an ambient air temperature or a temperature of the interior of the cassette (1) or a difference of the ambient air temperature and the temperature of the interior of the cassette (1).

6. Medicament delivery system according to any one of claims 1 to 5, wherein the pump unit (2) comprises a processing unit (4) and wherein the processing unit (4) is configured to control the pump (3) and the air valve (3) to periodically pressurise and de-pressurise the interior of the cassette (1),
wherein the processing unit (4) is preferably configured to perform the periodic pressurising and de-pressurising based on an ambient air temperature or a temperature of the interior of the cassette (1) or a difference of the ambient air temperature and the temperature of the interior of the cassette (1).

7. Medicament delivery system of any one of claims 1 to 6, wherein the pump unit (2) is independent from said cassette (1).

8. Medicament delivery system according to any one of claims 1 to 7, wherein the cassette (1) comprises an entry passage (14) configured to communicate with a connector (32) of the pump unit (2),
wherein the pump (3) is configured to pressurise the cassette (1) via the connector (32) and the entry passage (14).

9. Medicament delivery system according to any one of claims 1 to 8, wherein the cassette (1) comprises at least one outer shell (11) and a lid (17), wherein the entry passage (14) is preferably provided in the lid (17), and
wherein the medicament bag (12) is preferably provided inside the at least one outer shell (11) and the lid (17).

10. Medicament delivery system according to any one of claims 1 to 9, wherein the medicament bag (12) is a flexible medicament bag (12).

11. Method for warming a medicament in a medicament delivery system, according to any one of claims 1 to 10, wherein the method comprises:
pressurising (T3) a cassette (1) containing a medicament bag (12),
releasing (T4), after a predetermined period of time, the pressurised air from the cassette (1).

12. Method according to claim 11, further comprising measuring (T5) an outflow temperature of the released air.

13. Method according to claim 12, further comprising repeating the pressurising (T3) and releasing (T4) step depending on the measured outflow temperature.

14. Method according to claim 12 or 13, further comprising measuring an inflow temperature and calculating a temperature difference between the inflow temperature and the outflow temperature.

15. Method according to claim 14, further comprising repeating the pressurising (T3) and releasing (T4) step depending on the measured temperature difference.

## Patentansprüche

1. Arzneimittelabgabesystem, umfassend:
eine Kassette (1), umfassend einen Arzneimittelbeutel (12) in einem Inneren davon,
eine Pumpeneinheit (2), die von der Kassette (1) trennbar ist, wobei eine Luftströmungsverbindung zwischen der Pumpeneinheit (2) und dem Inneren der Kassette (1) bereitgestellt ist,
wobei die Pumpeneinheit (2) eine Pumpe (3) und ein Luftventil (31) umfasst,
wobei die Pumpe (3) konfiguriert ist, um das Innere der Kassette (1) mit Druck zu beaufschlagen, um einen Druck auf den Arzneimittelbeutel (12) auszuüben, und
wobei das Luftventil (31) konfiguriert ist, um die Kassette (1) durch Ablassen des Drucks aus dem Inneren der Kassette (1) drucklos zu machen
**dadurch gekennzeichnet, dass** die Pumpeneinheit (2) mindestens einen Temperatursensor umfasst, wobei der mindestens eine Temperatursensor in einem Luftströmungsweg an der Pumpe (3) und/oder dem Luftventil (31) und/oder einem Luftfilter (35), der mit der Pumpe (3) verbunden ist, bereitgestellt ist.

2. Arzneimittelabgabesystem nach Anspruch 1, wobei die Pumpeneinheit (2) mindestens einen Drucksensor umfasst, wobei der mindestens eine Drucksensor bevorzugt in einem Luftströmungsweg an der Pumpe (3) und/oder dem Luftventil (31) und/oder einem Luftfilter (35), der mit der Pumpe (3) verbunden ist, bereitgestellt ist.

3. Arzneimittelabgabesystem nach Anspruch 1, wobei der mindestens eine Temperatursensor konfiguriert ist, um mindestens eine von einer Umgebungslufttemperatur und einer Temperatur des Inneren der Kassette (1) zu messen.

4. Arzneimittelabgabesystem nach Anspruch 1, 2 oder 3, wobei der mindestens eine Drucksensor konfiguriert ist/sind, um den Druck des Inneren der Kassette (1) zu messen.

5. Arzneimittelabgabesystem nach einem der Ansprüche 1 bis 4, wobei die Pumpeneinheit (2) eine Verarbeitungseinheit (4) umfasst, und wobei die Verarbeitungseinheit (4) konfiguriert ist, um die Pumpe (3) basierend auf einer Umgebungslufttemperatur oder einer Temperatur des Inneren der Kassette (1) oder einer Differenz der Umgebungslufttemperatur und der Temperatur des Inneren der Kassette (1) zu steuern.

6. Arzneimittelabgabesystem nach einem der Ansprüche 1 bis 5, wobei die Pumpeinheit (2) eine Verarbeitungseinheit (4) umfasst, und wobei die Verarbeitungseinheit (4) konfiguriert ist, um die Pumpe (3) und das Luftventil (3) zu steuern, um das Innere der Kassette (1) periodisch mit Druck zu beaufschlagen und drucklos zu machen,
wobei die Verarbeitungseinheit (4) vorzugsweise konfiguriert ist, um das periodische Druckbeaufschlagen und Drucklosmachen basierend auf einer Umgebungslufttemperatur oder einer Temperatur des Inneren der Kassette (1) oder einer Differenz der Umgebungslufttemperatur und der Temperatur des Inneren der Kassette (1) durchzuführen.

7. Arzneimittelabgabesystem nach einem der Ansprüche 1 bis 6, wobei die Pumpeneinheit (2) von der Kassette (1) unabhängig ist.

8. Arzneimittelabgabesystem nach einem der Ansprüche 1 bis 7, wobei die Kassette (1) einen Eintrittsdurchgang (14), der konfiguriert ist, um mit einem Verbinder (32) der Pumpeneinheit (2) zu kommunizieren, umfasst,
wobei die Pumpe (3) konfiguriert ist, um die Kassette (1) über den Verbinder (32) und den Eintrittsdurchgang (14) mit Druck zu beaufschlagen.

9. Arzneimittelabgabesystem nach einem der Ansprüche 1 bis 8, wobei die Kassette (1) mindestens eine Außenhülle (11) und einen Deckel (17) umfasst, wobei der Eintrittsdurchgang (14) vorzugsweise in dem Deckel (17) bereitgestellt ist, und
wobei der Arzneimittelbeutel (12) vorzugsweise innerhalb der mindestens einen Außenhülle (11) und des Deckels (17) bereitgestellt ist.

10. Arzneimittelabgabesystem nach einem der Ansprüche 1 bis 9, wobei der Arzneimittelbeutel (12) ein flexibler Arzneimittelbeutel (12) ist.

11. Verfahren zum Erwärmen eines Arzneimittels in einem Arzneimittelabgabesystem nach einem der Ansprüche 1 bis 10, wobei das Verfahren umfasst:
Druckbeaufschlagen (T3) einer Kassette (1), die einen Arzneimittelbeutel (12) enthält,
Ablassen (T4), nach einem zuvor bestimmten Zeitraum, der Druckluft aus der Kassette (1).

12. Verfahren nach Anspruch 11, ferner umfassend das Messen (T5) einer Ausströmungstemperatur der abgelassenen Luft.

13. Verfahren nach Anspruch 12, ferner umfassend ein Wiederholen des Schritts des Druckbeaufschlagens (T3) und Ablassens (T4) abhängig von der gemessenen Ausströmungstemperatur.

14. Verfahren nach Anspruch 12 oder 13, ferner umfassend das Messen einer Einströmungstemperatur und ein Berechnen einer Temperaturdifferenz zwischen der Einströmungstemperatur und der Ausströmungstemperatur.

15. Verfahren nach Anspruch 14, ferner umfassend das Wiederholen des Schritts des Druckbeaufschlagens (T3) und Ablassens (T4) abhängig von der gemessenen Temperaturdifferenz.

## Revendications

1. Dispositif d'administration de médicament comprenant :
une cassette (1) comprenant une poche à médicament (12) en son intérieur,
une unité pompe (2) séparable de la cassette (1), dans lequel un raccordement d'écoulement d'air est prévu entre l'unité pompe (2) et l'intérieur de la cassette (1),
dans lequel l'unité pompe (2) comprend une pompe (3) et une soupape à air (31),
dans lequel la pompe (3) est conçue pour mettre sous pression l'intérieur de la cassette (1) afin d'exercer une pression sur la poche à médicament (12) et
dans lequel la soupape à air (31) est conçue pour dépressuriser la cassette (1) par relâchement de la pression de l'intérieur de la cassette (1)
**caractérisé en ce que** l'unité pompe (2) comprend au moins un capteur de température, dans lequel l'au moins un capteur de température est disposé dans un trajet d'écoulement d'air au niveau de la pompe (3) et/ou de la soupape à air (31) et/ou d'un filtre à air (35) raccordé à la pompe (3).

2. Système d'administration de médicament selon la revendication 1, dans lequel l'unité pompe (2) comprend au moins un capteur de pression, dans lequel l'au moins un capteur de pression est de préférence disposé dans un trajet d'écoulement d'air au niveau de la pompe (3) et/ou de la soupape à air (31) et/ou d'un filtre à air (35) raccordé à la pompe (3).

3. Système d'administration de médicament selon la revendication 1, dans lequel l'au moins un capteur de température est configuré pour mesurer au moins une température parmi une température de l'air ambiant et une température de l'intérieur de la cassette (1).

4. Système d'administration de médicament selon la revendication 1, 2 ou 3, dans lequel l'au moins un capteur de pression est configuré pour mesurer la pression à l'intérieur de la cassette (1).

5. Système d'administration de médicament selon l'une quelconque des revendications 1 à 4, dans lequel l'unité pompe (2) comprend une unité de traitement (4) et dans lequel l'unité de traitement (4) est configurée pour commander la pompe (3) sur la base d'une température de l'air ambiant ou d'une température à l'intérieur de la cassette (1) ou d'une différence entre la température de l'air ambiant et la température à l'intérieur de la cassette (1).

6. Système d'administration de médicament selon l'une quelconque des revendications 1 à 5, dans lequel l'unité pompe (2) comprend une unité de traitement (4) et dans lequel l'unité de traitement (4) est configurée pour commander la pompe (3) et la soupape à air (3) afin de mettre sous pression et de dépressuriser périodiquement l'intérieur de la cassette (1),
dans lequel l'unité de traitement (4) est de préférence configurée pour effectuer la mise sous pression et la dépressurisation périodiques sur la base d'une température de l'air ambiant ou d'une température à l'intérieur de la cassette (1) ou d'une différence entre la température de l'air ambiant et la température à l'intérieur de la cassette (1).

7. Système d'administration de médicament selon l'une quelconque des revendications 1 à 6, dans lequel l'unité pompe (2) est indépendante de ladite cassette (1).

8. Système d'administration de médicament selon l'une quelconque des revendications 1 à 7, dans lequel la cassette (1) comprend un passage d'entrée (14) conçu pour communiquer avec un raccord (32) de l'unité pompe (2),
dans lequel la pompe (3) est conçue pour mettre sous pression la cassette (1) par l'intermédiaire du raccord (32) et du passage d'entrée (14).

9. Système d'administration de médicament selon l'une quelconque des revendications 1 à 8, dans lequel la cassette (1) comprend au moins une enveloppe externe (11) et un couvercle (17), dans lequel le passage d'entrée (14) est de préférence disposé dans le couvercle (17) et
dans lequel la poche à médicament (12) est de préférence disposée à l'intérieur de l'au moins une enveloppe externe (11) et du couvercle (17).

10. Système d'administration de médicament selon l'une quelconque des revendications 1 à 9, dans lequel la poche à médicament (12) est une poche à médicament (12) souple.

11. Procédé destiné à réchauffer un médicament dans un système d'administration de médicament selon l'une quelconque des revendications 1 à 10, dans lequel le procédé comprend :
la mise sous pression (T3) d'une cassette (1) contenant une poche à médicament (12),
le relâchement (T4), après une période de temps prédéterminée, de l'air sous pression de la cassette (1).

12. Procédé selon la revendication 11, comprenant en outre la mesure (T5) d'une température de sortie de l'air relâché.

13. Procédé selon la revendication 12, comprenant en outre la répétition de l'étape de mise sous pression (T3) et l'étape de relâchement (T4) en fonction de la température de sortie mesurée.

14. Procédé selon la revendication 12 ou 13, comprenant en outre la mesure d'une température d'entrée et le calcul d'une différence de température entre la température d'entrée et la température de sortie.

15. Procédé selon la revendication 14, comprenant en outre la répétition de l'étape de mise sous pression (T3) et l'étape de relâchement (T4) en fonction de la différence de température mesurée.
